Europäisches Patentamt

European Patent Office

Office européen des brevets

(11) Publication number: **0 520 408 A2**

# EUROPEAN PATENT APPLICATION

(21) Application number: **92110640.7**

(22) Date of filing: **24.06.92**

(51) Int. Cl.⁵: **A61B 10/00**

(30) Priority: **25.06.91 US 722333**
**03.06.92 US 889283**

(43) Date of publication of application:
**30.12.92 Bulletin 92/53**

(84) Designated Contracting States:
**DE GB IT NL SE**

(71) Applicant: **SALIVA DIAGNOSTIC SYSTEMS, INC.**
**11719 N.E. 95th Street, Suite G**
**Vancouver, Washington 98682(US)**

(72) Inventor: **Seymour, Eugene H., Dr.**
**1465 Monaco Drive**
**Pacific Palisades, California 90272(US)**

(74) Representative: **Patentanwälte Grünecker, Kinkeldey, Stockmair & Partner**
**Maximilianstrasse 58**
**W-8000 München 22(DE)**

(54) **Sampling device and sample adequacy system.**

(57) A sampling device (210, 1710, 1910, 2110, 2310) which can be used to safely obtain samples of biological fluids, such as, for example, saliva, urine, water, tears, and vaginal fluids. The device can include a sample container (211, 1711, 1911, 2111, 2311), a sample collector for collecting a sample of biological fluids (213, 1713, 2113, 2313), and a collection container (1714, 1914, 2155). Means (214, 1715, 1915, and 2114) can be provided, so that when the sample collector selectively receives a sample of biological fluid, an indicator activated by a preselected amount of the received sample of biological fluid indicates that the collected sample of biological fluid is of adequate amount. The sample collector may, for example, include a piston (1932, 2115) fitting closely within the sample container and a porous mass which may be compressed by the piston in the sample container to extract the sample of biological fluids. For example, when the sample container (1911) is inserted into the collection container (1914) the sample container (1911) becomes fluidly coupled to the collection container (1914) which receives the collected sample of biological fluids. In another embodiment, a sampling device (2110) may also include an assembly with at least one container (2170) containing a solution which may be used to mix with the collected sample of biological fluids.

FIG. 1

EP 0 520 408 A2

The field of the invention is devices for sampling biological fluids.

The current literature indicates saliva is used to conveniently, easily, safely and effectively test an individual for a variety of medical conditions. These tests for medical conditions include a hepatitis screening for restaurant employees, HIV, continue (nicotine) and cocaine screening by insurance companies, and an HIV antibody screening for medical purposes. Clinics for oncology, neurology, infertility, allergy, orthopedics and pain which had used urine, blood and serum samples to determine the medical conditions of their patients are now using samples of saliva for this same purpose.

The present invention is generally directed to a sampling device which includes a sample container, a sample collector and a collection container for use in collecting biological fluids.

In a first aspect of the present invention, the sample collector is sized to fit through the opening of the sample container. The sample container has an opening at a first end and a passageway through a second end. There is a selective closure in the passageway. The collection container has an opening which is sized so that the second end of the sample container fits within it.

In a second aspect of the present invention, the selective closure includes a closure element blocking the passageway and an actuator element extending from the collection container upwardly toward the passageway of the sample container to contact and interfere with the selective closure, so that upon insertion of the sample container in said collection container said closure element is displaced and the passageway is opened.

In a third aspect of the present invention, the sample collector includes a piston fitting closely within the sample container and a porous mass which may be compressed by the piston in the sample container to extract the sample of biological fluids.

In a fourth aspect of the present invention, the sampling device also includes an assembly with at least one container containing a solution which may be used to mix with the collected sample of biological fluids.

In a fifth aspect of the present invention, the sampling device includes a solution contained in the sample container and a label attached thereto. The bottom edge of the label is disposed adjacent to the liquid level of the solution. When the sample collector is placed in the sample container, if the liquid level of the solution does not drop below the bottom edge of the label then the collected sample is of an adequate amount.

In a sixth aspect of the present invention, when the sample collector selectively receives a sample of biological fluid an indicator activated by a preselected amount of the received sample of biological fluid indicates that the collected sample of biological fluid is of an adequate amount.

Other aspects and many of the attendant advantages will be more readily appreciated as the same becomes better understood by reference to the following detailed description and considered in connection with the accompanying drawing in which like reference symbols designate like parts throughout the figures.

The features of the present invention which are believed to be novel are set forth with particularity in the appended claims.

Figure 1 is a perspective view of a sample container of a saliva sampling device in accordance with the first embodiment.

Figure 2 is a perspective view of a saliva collector including a holder, an elongated member and a piece of filter paper.

Figure 3 is a side elevational view in cross-section of the saliva collector of Figure 2 before the saliva collector has been placed in a subject's mouth.

Figure 4 is a side elevational view in cross-section of the saliva collector of Figure 6 after the saliva collector has been placed in a subject's mouth, but before it has collected an adequate sample of saliva.

Figure 5 is a side elevational view in cross-section of the saliva collector of Figure 2 after the saliva collector has collected an adequate sample of saliva.

Figure 6 is a perspective view of the sample container of Figure 1 and the saliva collector of Figure 2 after the saliva collector has collected an adequate sample of saliva.

Figure 7 is a perspective view of a sample container of a saliva sampling device with a squeeze cap, which is in the closed position, in accordance with the second embodiment.

Figure 8 is a perspective view of a saliva collector of the saliva sampling device of Figure 7 which includes a holder, an elongated member, a piece of filter paper and a sample adequacy system.

Figure 9 is a perspective view of a collection container of the saliva sampling device of Figure 7.

Figure 10 is an elevational view in a partial cross-section of the sample adequacy system of the saliva collector of Figure 8.

Figure 11 is a cross-sectional view of the squeeze cap of Figure 7 which is in the open position.

Figure 12 is an elevational view in cross-section of the sample container of Figure 7 with the sample container including a breakable seal and containing a chemical solution, such as, for example, a buffering solution.

Figure 13 is an elevational view in cross-section of the collection container of Figure 9.

Figure 14 is an elevational view in cross-section of the sample container of Figure 7 after the cap has been removed from the sample container, the sample container has been placed in the collection container of Figure 9 and the saliva collector of Figure 8, which has collected an adequate sample of saliva, has been inserted into the sample container, but before the saliva collector has been pressed downward, so that the sample of saliva is mixed with the buffering solution.

Figure 15 is an elevational view in cross-section of the sample container of Figure 7 after the saliva collector of Figure 8 has been pressed downward into the collection container of Figure 9 to break the breakable seal thereof in order to release the mixture of the buffering solution and the saliva so that this mixture flows into the collection container.

Figure 16 is an elevational view in cross-section of the collection container of Figure 9 which has the mixture of the buffering solution and the saliva contained therein and which is sealed by the squeeze cap of Figure 7 in the closed position.

Figure 17 is a perspective view of a sample container of a sampling device in accordance with the third embodiment.

Figure 18 is a perspective view of a sample collector of the sampling device of Figure 17 including a sample adequacy system.

Figure 19 is a perspective view of an analysis container of the sampling device of Figure 17.

Figure 20 is an elevational view in a partial cross-section of the sample adequacy system of Figure 18 before the sample collector of Figure 18 it has collected a sample.

Figure 21 is an elevational view of the sample adequacy system of Figure 18 after the sample collector has collected an adequate sample.

Figure 22 is an elevational view in cross-section of the sample container of Figure 17 which has a breakable seal and is filled with a buffering solution.

Figure 23 is an elevational view in cross-section of the analysis container of Figure 19 which has a reagent pad. Figure 24 is an elevational view in cross-section of the sample container of Figure 17, the sample collector of Figure 18 and the analysis container of Figure 19 after the sample collector has collected an adequate sample and it has been inserted into the sample container so that the sample mixes with the buffering solution within the sample container and after the sample container has been inserted into the analysis container.

Figure 25 is an elevational view in cross-section of the sample container of Figure 17, the sample collector of Figure 18 and the analysis container of Figure 19 after the sample collector of Figure 18 has been pressed downward into the analysis container to break the breakable seal of the sample container thereby releasing the mixture of the buffering solution and the sample so that this mixture flows into the analysis container.

Figure 26 is a top plan view in cross-section of the analysis container of Figure 19 in which has the mixture of the buffering solution and the sample has flowed onto the reagent pad to be absorbed thereby taken along the line 26-26 of Figure 23.

Figure 27 is a perspective view of a sample container of a sampling device including a sample adequacy system in accordance with the fourth embodiment.

Figure 28 is a perspective view of a sample collector of the sampling device of Figure 27.

Figure 29 is an elevational view in a partial cross-section of the sample adequacy system of the sample collector of Figure 28 before it has collected a sample.

Figure 30 is an elevational view of the sample adequacy system of Figure 29 after the sample collector has collected an adequate sample.

Figure 31 is an elevational view in cross-section of the sample container of Figure 27 including a breakable seal and containing a buffering solution.

Figure 32 is a perspective view of an assembly of the sampling device of Figure 27 including a lower cap, an upper cap and a sleeve.

Figure 33 is a front elevational view in cross-section of the lower cap of Figure 32.

Figure 34 is a side elevational view in cross-section of the lower cap of Figure 32.

Figure 35 is a top plan view of the lower cap of Figure 32.

Figure 36 is a cross-sectional view of the lower cap of Figure 32 taken along line 36-36 of Figure 34.

Figure 37 is a perspective view of the upper cap of Figure 32.

Figure 38 is an elevational view in cross-section of the upper cap of Figure 32.

Figure 39 is a cross-sectional view of the upper cap of Figure 32 taken along line 39-39 of Figure 38.

Figure 40 is a perspective view of the sleeve of Figure 32.

Figure 41 is a cross-sectional view of the sleeve of Figure 32 taken along line 41-41 of Figure 43.

Figure 42 is a bottom plan view of the sleeve of Figure 32.

Figure 43 is a cross-sectional view of the sleeve of Figure 32 taken along the line 43-43 of Figure 41.

Figure 44 is a perspective view of a carrier of the assembly of Figure 32.

Figure 45 is an elevational view in cross-section of the carrier of Figure 44.

Figure 46 is a cross-sectional view of the carrier of Figure 44 taken along the line 46-46 of Figure 45.

Figure 47 is a plan view of a test strip which has six segments before it has been wrapped for use in the assembly of Figure 32.

Figure 48 is an elevational view of the test strip of Figure 47 after it has been wrapped, but before it has been inserted between the carrier and the sleeve of the assembly of Figure 32.

Figure 49 is a top plan view of the test strip of Figure 48.

Figure 50 is an elevational view in cross-section of the upper cap which has a first capsule and a second capsule disposed therein, the sleeve and the carrier of the assembly of Figure 34 the sample container of Figure 27 and the sample collector of Figure 28, after the carrier has been inserted into the sleeve, the sample container has been inserted into the carrier and the sample collector has been inserted into the sample container so that the sample is able to mix with the buffering solution, the sample collector is ready to be pressed downward.

Figure 51 is an elevational view in cross-section of the sample container of Figure 27, the sample collector of Figure 28 and the assembly of Figure 32, after the sample collector has been pressed downward and the breakable seal of the sample container has been broken thereby having released the mixture of the buffering solution and the sample so that this mixture flows into the upper cap of the assembly.

Figure 52 is an enlarged partial elevational view in cross-section of the sleeve, the carrier and the lower cap of the assembly of Figure 32 at circle 52 of Figure 34.

Figure 53 is an enlarged, partial elevational view in cross-section of the sample container of Figure 27, the sample collector of Figure 28 and the assembly of Figure 32 after the sample collector of Figure 28 has been pressed downward.

Figure 54 is an elevational view of the sample container of Figure 27, the sample collector of Figure 28 and the assembly of Figure 32, after the sample container, the sample collector and the assembly have been turned over in order to allow the mixture of the buffering solution and the sample to flow onto the six segments of the test strip so that the mixture of the buffering solution and the sample is on each segment.

Figure 55 is an elevational view in cross-section of the sample container of Figure 25, the sample collector of Figure 28 and the assembly of Figure 32 after the sample container, the sample collector and the assembly have been turned over.

Figure 56 is a cross-sectional view of the sample container of Figure 27 and the assembly of Figure 32 taken along line 56-56 of Figure 55 after the sample container, the sample collector and the assembly have been turned over.

Figure 57 is an enlarged, partial elevational view in cross-section of the sample container of Figure 27 and the assembly of Figure 32 after the sample container, the sample collector and the assembly have been turned over.

Figure 58 is a cross-sectional view of the sample container of Figure 27 and the assembly of Figure 32 taken along line 58-58 of Figure 55 after the first and second capsules of Figure 50 have been broken in order to allow the developer liquid to flow onto the six segments of the test strip of Figure 47 so that the developer liquid mixes with the mixture of the buffering solution and the sample on each segment.

Figure 59 is an enlarged, partial elevational view in cross-section of the sample container of Figure 27 and the assembly of Figure 32 after the first and second capsules of Figure 47 have been broken.

Figure 60 is a perspective view of an upper cap of an assembly of a sampling device.

Figure 61 is a top plan view in cross-section of the assembly of Figure 60 which has a first capsule and a second capsule, after the assembly have been turned over in order to allow the mixture of the buffering solution and the sample to flow onto the six segments of the test strip so that the mixture of the buffering solution and the sample is on each segment.

Figure 62 is an elevational view in cross-section of the sample container of Figure 27 and the assembly of Figure 60 taken along line 62-62 of Figure 61 after the sample container and the assembly have been turned over.

Figure 63 is a top plan view in cross-section of the assembly of Figure 60 after the first capsule of Figure 61, which contains a first developer liquid, has been broken in order to allow the first developer liquid to flow onto the six segments of the test strip of Figure 47 into the mixture of the buffering solution and the sample so that the first developer liquid mixes with the mixture of the buffering solution and the sample on each segment.

Figure 64 is an elevational view in cross-section of the sample container of Figure 27 and the assembly of Figure 60 taken along line 64-64 of Figure 63 after the first capsule of Figure 61 has been broken.

Figure 65 is a top plan view in cross-section of the assembly of Figure 60 after the second capsule of Figure 61, which contains a first developer liquid, has been broken in order to allow the second developer liquid to flow onto the six segments of the test strip of Figure 47 into the mixture of the first developer liquid, the buffering solution and the sample so that the second developer liquid mixes with the mixture of the first developer liquid, the buffering solution and the sample on each segment.

Figure 66 is an elevational view in cross-section of the sample container of Figure 27 and the assembly of Figure 60 taken along line 66-66 of Figure 65 after the second capsule of Figure 61 has been broken.

Figure 67 is a plan view of a test strip which has six segments one of which is a timing strip for use with the first and second developer liquids of Figure 63 and Figure 65 respectively, before the test strip has been wrapped for use in the assembly of Figure 60.

Figure 68 is a perspective view of a sampling device including a sample container and a cap.

Figure 69 is a side view in cross-section of a fingerprint sensitive pad.

Figure 70 is a partial front elevational view of the sampling device of Figure 68 after the fingerprint sensitive pad of Figure 69 has been adhesively coupled to both the sample container and the cap.

Figure 71 is a perspective view of a sample container of a sampling device for either stool or soil in accordance with the seventh embodiment.

Figure 72 is a perspective view of a sample container of a sampling device for blood.

Figure 73 is a perspective view of the sample container of Figure 72 when it is filled with blood.

## DESCRIPTION OF THE PREFERRED EMBODIMENTS

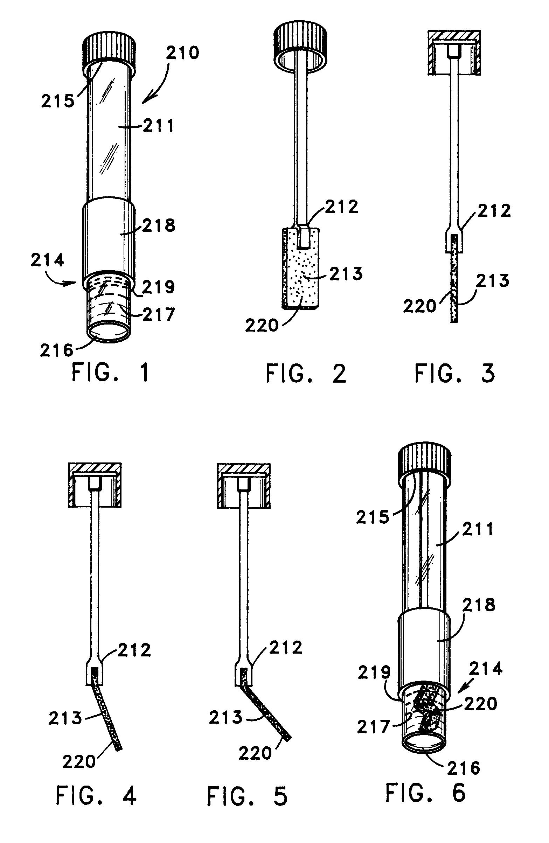

Referring to Figure 1 in conjunction with Figure 2 and Figure 3 a saliva sampling device 210 includes a sample container 211, a holder 212, a saliva collector 213 and a sample adequacy system 214. The sample container 211 has an open end 215 and a closed end 216. The sample adequacy system includes a solution 217 which is contained in the sample container 211 at a liquid level and a label 218 which has a bottom edge and which is attached to the sample container 211. The bottom edge 219 of the label 218 is disposed adjacent to the liquid level of the solution 217. The saliva collector 213 is a piece of filter paper 220 which is fixed to the holder 212. The saliva collector 213 collects a sample of saliva. The sample adequacy system 214 determines that the collected sample of saliva is an adequate sample when the saliva collector 213 is placed in the sample container 211 and the liquid level of the solution 217 does not drop below the bottom edge 219 of the label 218.

Referring to Figure 2 in conjunction with Figure 3 the saliva collector 213 is shown before the saliva collector 213 has been placed in a subject's mouth.

Referring to Figure 4 the saliva collector 213 is shown after the saliva collector 213 has been placed in a subject's mouth, but before it has collected an adequate sample of saliva.

Referring to Figure 5 the saliva collector 213 is shown after the saliva collector 213 has collected an adequate sample of saliva.

Referring to Figure 6, the saliva collector 213 has collected an adequate sample of saliva when the saliva collector 213 is placed in the sample container 211 and the liquid level of the solution 217 does not drop below the bottom edge 219 of the label 218. If the liquid level of the solution 217 drops below the bottom edge 219 of the label 218 then the sample of saliva is not adequate.

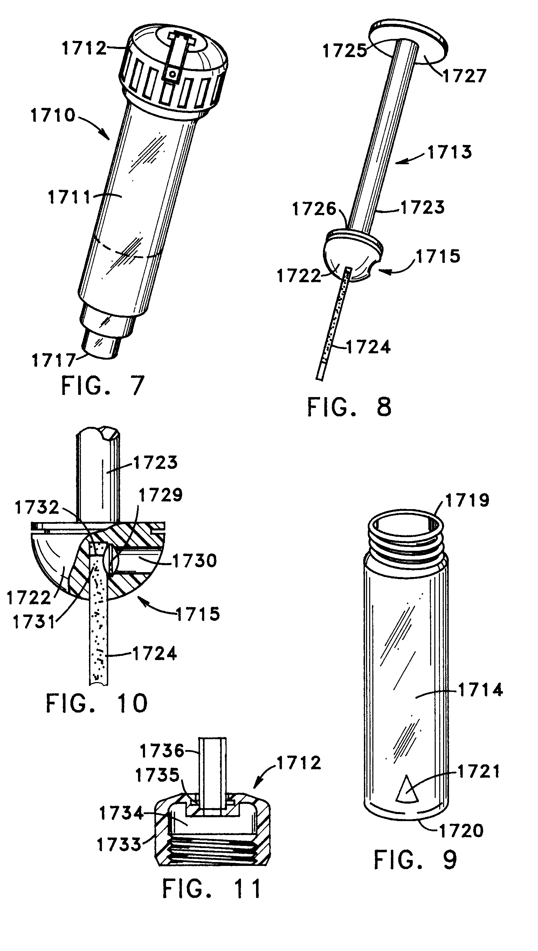

Referring to Figure 7 in conjunction with Figure 8 and Figure 9 a saliva sampling device 1710 includes a sample container 1711 having a squeeze cap 1712, a saliva collector 1713, a collection container 1714 and a sample adequacy system 1715. The sample container 1711 has an open end 1716 and a closed end 1717. The sample container 1711 has a breakable seal 1718 which is disposed at the closed end 1717. The collection container 1714 has an open end 1719, which the squeeze cap 1712 is adapted to close, and a closed end 1720. The cap 1712 seals the sample container 1711 air-tight. The collection container 1714 has a seal-breaking member 1721 disposed at the closed end thereof. The saliva collector 1713 includes a holder 1722, an elongated member 1723 and a piece of filter paper 1724. The elongated member 1723 has a first end 1725 and a second end 1726. The first end 1725 of the elongated member 1724 is coupled to a push tab 1727. The holder 1722 is coupled to the second end 1726 of the elongated member 1723. The piece of filter paper 1724 is of predetermined dimensions and is mechanically coupled to the holder 1722 so that a technician can collect a sample of saliva without touching the sample. The saliva collector 1713 selectively receives a sample of saliva.

Referring to Figure 8 in conjunction with Figure 10 the sample adequacy system 1715 includes a plastic lens 1729 and a hole 1730 in the top surface of the holder 1722 into which the plastic lens 1729 is disposed. The top portion 1731 of the piece of filter paper 1724 is treated with a chemical reagent 1732 which reacts with saliva by changing its color from a first color, e.g. either clear or blue, to a second color, e.g. either blue or clear. Before the saliva collector 1713 has been placed in a subject's mouth the top portion 1731 of the piece of filter paper 1724 is of the color clear. When an adequate amount of saliva has been collected the saliva in the piece of filter paper 1724 will reach the chemical reagent 1732 and change from clear to the color blue. In other embodiments the sample adequacy system 1715 includes either a compressed sponge or an expandable polymeric beam which expands which it comes in contact with saliva and which is disposed in the top portion 1731 of the piece of filter paper 1724. When the saliva collector 1713 has collected an adequate sample, the saliva will reach either the compressed sponge or the expandable polymeric bead and expand it.

Referring to Figure 7 in conjunction with Figure 11 the squeeze cap 1712 includes a cap 1733 with a bore 1734, a pivot mount 1735 and a spout 1736 which is pivotally coupled to the pivot mount 1735. The spout 1736 is shown in the open position.

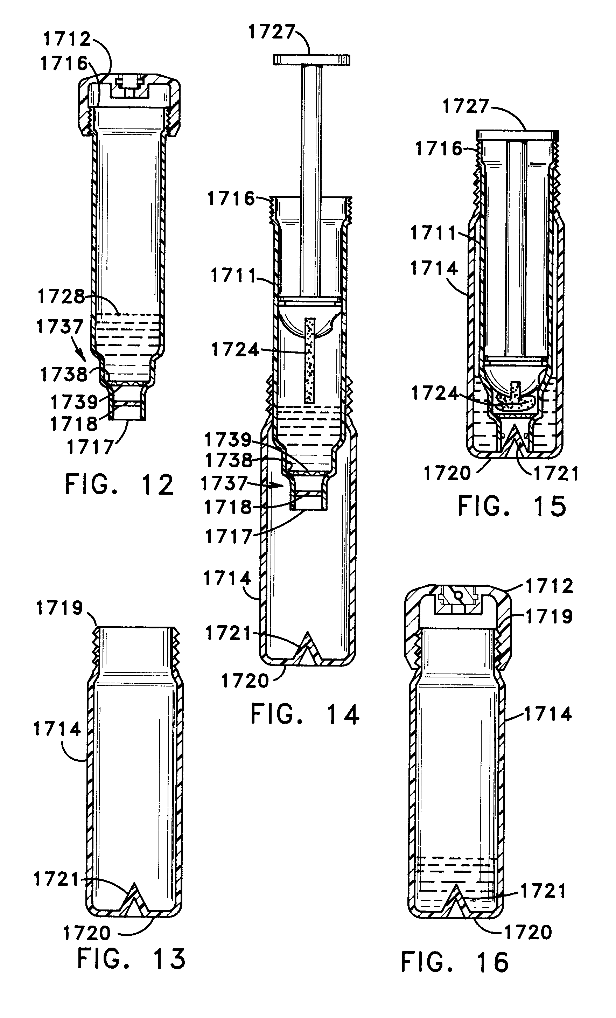

Referring to Figure 7 in conjunction with Figure 8 and Figure 12, a chemical solution, such as, for example, a buffering solution 1728 is contained in the sample container 1711. In another embodiment the sample container 1711 may be formed out of a non-opaque plastic material. The sample adequacy system 1715 may then include a label having a bottom edge attached to the sample container 1711. The bottom edge of the label is disposed adjacent to the liquid level of the buffering solution 1728. When the saliva collector 1713 is placed in the sample container 1711, if the liquid level of the buffering solution 1728 does not drop below the bottom edge of the label then the collected sample of saliva is of an adequate amount.

Referring to Figure 7 in conjunction with Figure 12, Figure 13, Figure 14, Figure 15 and Figure 16 the sample container 1711 also includes a filtering system 1737 including a rubber o-ring 1738 and a piece of filtering material 1739. The filtering system 1737 is disposed adjacent to the breakable seal 1718. Once an adequate amount of saliva has been collected the saliva the saliva collector 1713 is inserted into the sample container 1711, after the squeeze cap 1712 has been removed therefrom, in order to mix the buffering solution 1728 and the sample of saliva together. When the sample container 1711 is inserted into the collection container 1714 and pressed downward the seal-breaking member 1721 breaks the breakable seal 1718 and the mixture of the buffering solution 1728 and the sample of saliva flows into the collection container 1714 through the filtering system 1737. The collection container 1714 has the mixture of the buffering solution 1728 and the saliva contained therein and is sealed by the squeeze cap 1712.

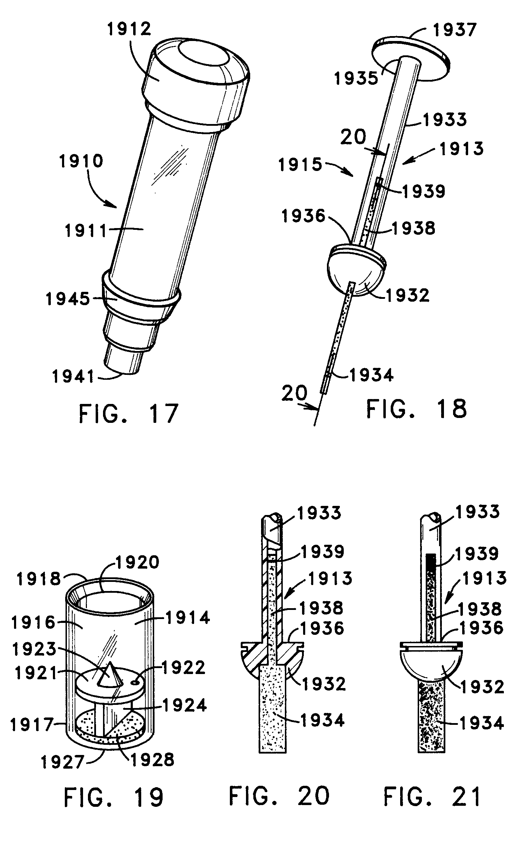

Referring to Figure 17 in conjunction with Figure 18 and Figure 19 a sampling device 1910 includes a sample container 1911 having a cap or means to seal 1912, a sample collector 1913, an analysis or collection container 1914 and a sample adequacy system 1915. The cap 1912 seals the sample container 1911 liquid-tight.

Referring to Figure 19 the analysis container 1914 includes a first cylinder 1916 and a second cylinder 1917. The first cylinder 1916 has a first open end 1918 and a second end 1919. The first cylinder 1916 includes an internal retaining ridge 1920 and a disc-shaped support 1921 with a drain hole 1922 therein. The internal retaining ridge 1920 is disposed at the first open end 1918 of the first cylinder 1916. The disc-shaped support 1921 has a seal-breaker 1923 and a divider 1924. The seal-breaker 1923 is mounted on the top surface of the disc-shaped support 1921. The divider 1924 is mounted on the bottom surface of the disc-shaped support 1921. The disc-shaped support 1921 is mechanically coupled to the first cylinder 1916 between the first and second open ends 1918 and 1919 thereof. The second cylinder 1917 has a first open end 1925 and a second end 1926. The second cylinder 1917 has a disc 1927 and a reagent pad 1928. The disc 1927 is mechanically coupled to the second cylinder 1917 at the second open end 1926 thereof in order to close the second open end 1926 thereof. After the reagent pad 1928 has been disposed on the disc 1927, the first and second cylinders 1916 and 1917 are joined by an adhesive 1929 to each other at their second and first open ends 1918 and 1925, respectively. When the first and second cylinders 1916 and 1917 are joined, the divider 1924 bisects the regent pad 1928 into a first portion 1930 and a second portion 1931 thereof. The first cylinder 1916 fluidly communicates with the first portion 1930 of the reagent pad 1928 in the second cylinder 1917 through the drain hole 1922. The first cylinder 1916 does not fluidly communicate with the second portion 1931 of the reagent pad 1928 in the second cylinder 1917. The first portion 1930 of the reagent pad 1928 fluidly communicates with the second portion 1931 thereof.

Referring to Figure 18 in conjunction with Figure 20 and Figure 21 the sample collector 1913 includes a piston-shaped holder 1932, an elongated member 1933 and a piece of filter paper 1934. The elongated member 1933 is formed out of a non-opaque plastic material and has a first end 1935 and a second end 1936. The first end 1935 of the elongated member 1933 is coupled to a push tab 1937. The piston-shaped holder 1932 is coupled to the second end 1936 of the elongated member 1933. The filter paper 1934 is of predetermined dimensions and is mechanically coupled to the piston-shaped holder 1932 so that a technician can collect a sample of sample without touching the sample. The sample adequacy system 1915 is formed by an extension 1938 of the filter paper 1934 and a either a drop of dye or a line of ink 1939 which is placed at the top of the extension 1938 of the filter paper 1934. When an adequate amount of sample has been collected the drop of dye 1939 mixes with a small amount of the collected sample causing the drop of dye 1939 to bleed into a larger area at the top of the extension 1938 of the filter paper 1934.

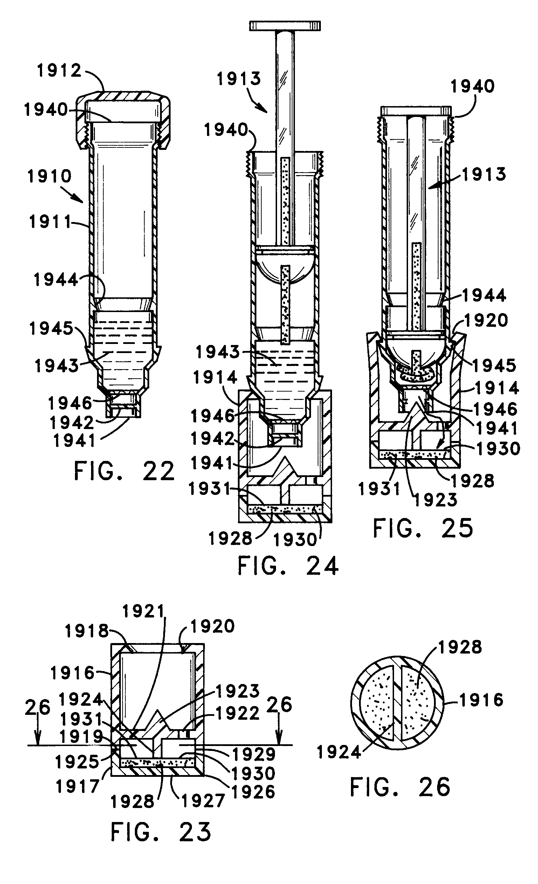

Referring to Figure 17 in conjunction with Figure 22 the sample container 1911 has an open end 1940 and a closed end 1941. The sample container 1911 has a breakable seal 1942 is disposed at the closed end 1941. A buffering solution 1943 is contained in the sample container 1911. The sample container 1911 also includes an internal retaining ridge 1944, an external retaining ridge 1945 and a filter 1946 which is formed out of filtering material. The filter 1946 is disposed adjacent to the breakable seal 1942.

Referring to Figure 24 in conjunction with Figure 21 when an adequate amount of sample has been collected, the sample collector 1913 is inserted into the sample container 1911 in order to mix the buffering solution 1943 and the sample together and the sample container 1911 is inserted into the analysis container 1914.

Referring to Figure 25 in conjunction with Figure 23, Figure 24 and Figure 26 when the sample collector 1913 has been inserted into the sample container 1911 and the sample container 1911 has been inserted into the analysis container 1914, the sample collector 1913 is pressed downward so that the seal-breaker 1923 breaks the breakable seal 1942 and the mixture of the buffering solution 1943 and the sample flows into the analysis container 1914 through the filter 1946 and onto the first portion 1930 of the reagent pad 1928 through the drain hole 1922. The internal retaining ridge 1944 of the sample container 1911 keeps the sample collector 1913 from being removed from the sample container 1911 by engaging the piston-shaped holder 1932. This prevents the sample collector 1913 from being used more than once. The external retaining ridge 1945 of the sample container 1911 serves as a means to secure the sample and analysis containers, as it keeps the sample container 1911 from being removed from the analysis container 1914 by engaging the internal retaining ridge 1920 of the analysis container 1914. This protects individuals from exposure to the sample of saliva contained in the analysis container 1914.

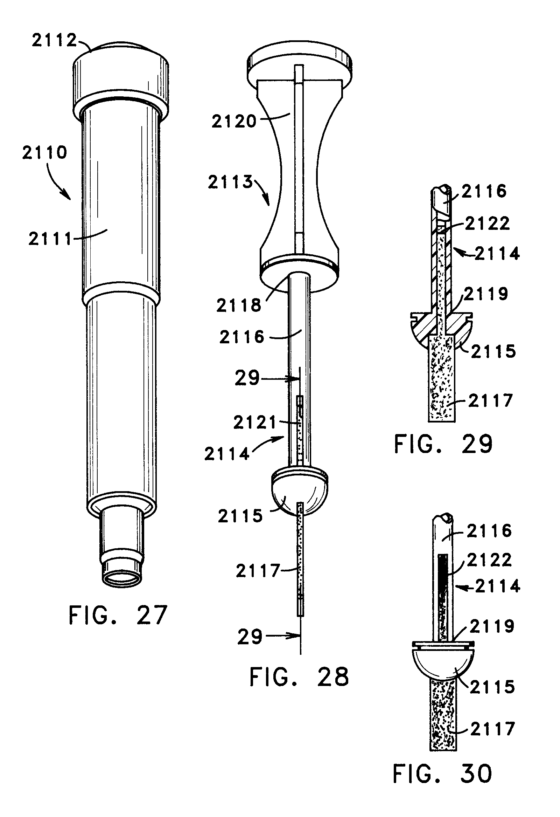

Referring to Figure 27 a sampling device 2110 includes a sample container 2111 having a cap 2112. The cap 2112 seals the sample container 2111 liquid-tight.

Referring to Figure 28 in conjunction with Figure 29 and Figure 30 the sampling device 2110 also includes a sample collector 2113 with a sample adequacy system 2114. The sample collector 2113 includes a piston-shaped holder 2115, an elongated member 2116 and a piece of filter paper 2117. The elongated member 2116 has a first end 2118 and a second end 2119. The first end 2118 of the elongated member 2116 is coupled to a push tab 2120. The piston-shaped holder 2115 is coupled to the second end 2119 of the elongated member 2116. The piece of filter paper 2117 is of predetermined dimensions and is mechanically coupled to the piston-shaped holder 2115 so that a technician can collect a sample without touching it. The sample adequacy system 2114 is formed by an extension 2121 of the filter paper 2117 and either a drop of dye 2122 or a line of ink placed at the top of the extension 2121 of the filter paper 2117. When an adequate amount of sample has been collected the drop of dye 2122 mixes with a small amount of the collected sample causing the drop of dye 2122 to bleed into a larger area at the top of the extension 2121 of the filter paper 2117. The sample collector 2113 selectively receives a sample. The sample collector 2113 can be used to collect samples of bodily fluids. These bodily fluids include saliva, urine, water, tears and vaginal fluids. The components of the sampling device 2110 may be formed out of a plastic material which may be either opaque or non-opaque.

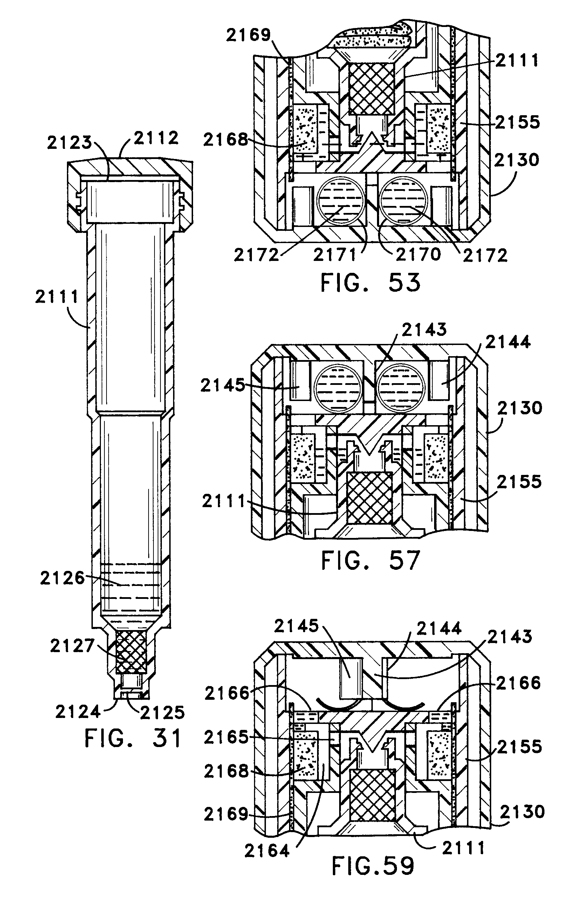

Referring to Figure 27 in conjunction with Figure 31 the sample container 2111 has an open end 2123 and a closed end 2124. The sample container 2111 has a breakable seal 2125 is disposed at the closed end 2124. A buffering solution 2126 is contained in the sample container 2111. The sample container 2111 also includes a filter 2127 which is formed out of filtering material. The filter 2127 is disposed adjacent to the breakable seal 2125.

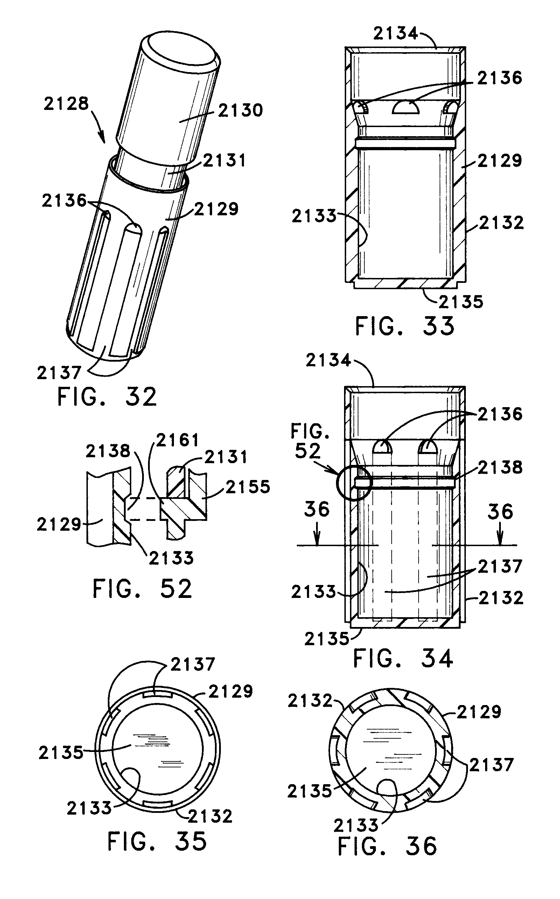

Referring to Figure 32 in conjunction with Figure 33, Figure 34, Figure 35 and Figure 36 an assembly 2128 includes a lower cap 2129, an upper cap 2130 and a sleeve 2131. The lower cap 2129 is hollow and substantially cylindrical. The lower cap 2129 has an outer surface 2132, an inner surface 2133, an open end 2134 and a closed end 2135. The lower cap 2129 also has six semi-circular holes 2136 adjacent to its open end 2134 and on its outer surface 2132 and six channels 2137 each of which runs from its closed end 2135 to one of the six semi-circular holes 2136. Each of the six channels 2137 is aligned with one of the six semi-circular holes 2136. The lower cap 2129 has a locking notch 2138 on its inner surface 2133.

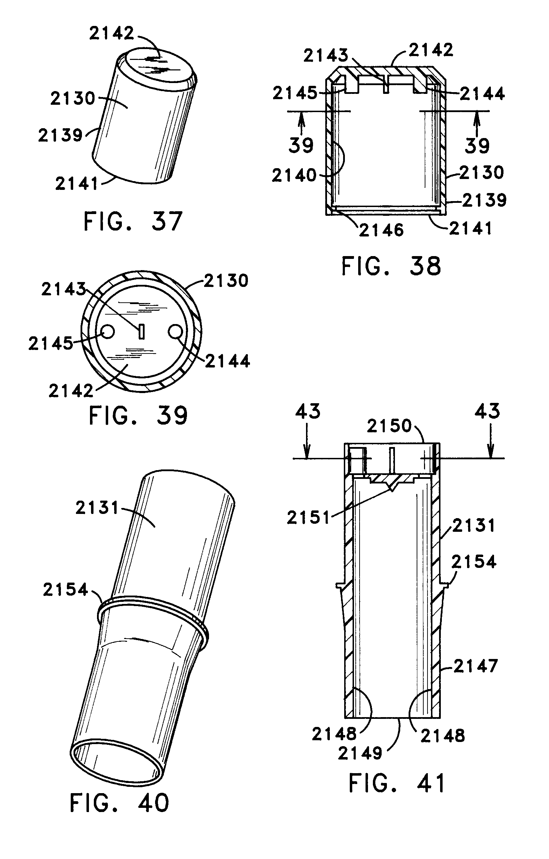

Referring to Figure 37 in conjunction with Figure 32, Figure 38 and Figure 39 the upper cap 2130 is hollow and substantially cylindrical. The upper cap 2130 has an outer surface 2139, an inner surface 2140, an open end 2141 and a closed end 2142. The upper cap 2130 also has a bar 2143, a first post 2144, a second post 2145 and an inner retaining ridge 2146. The bar 2143 is centrally disposed on the inner surface 2140 of the closed end 2142. The first and second posts 2144 and 2145 are oppositely disposed on the inner surface 2140 of the closed end 2142. The inner retaining ridge 2146 is disposed on the inner surface 2140 at the open end 2141.

Referring to Figure 40 in conjunction with Figure 32, Figure 41, Figure 42 and Figure 43 the sleeve 2131 is hollow and substantially cylindrical. The sleeve 2131 has an outer surface 2147, an inner surface 2148, an open end 2149 and a closed end 2150. The sleeve 2131 also has a seal-breaker 2151, four alignment bars 2152, six holes 2153 and a flange 2154. The four alignment bars 2152 and the six holes 2153 are disposed at the closed end 2142. The flange 2154 is disposed on the outer surface 2147. Each of the six holes 2153 may be aligned with one of the six semi-circular holes 2136 of the lower cap 2129 of the assembly 2128.

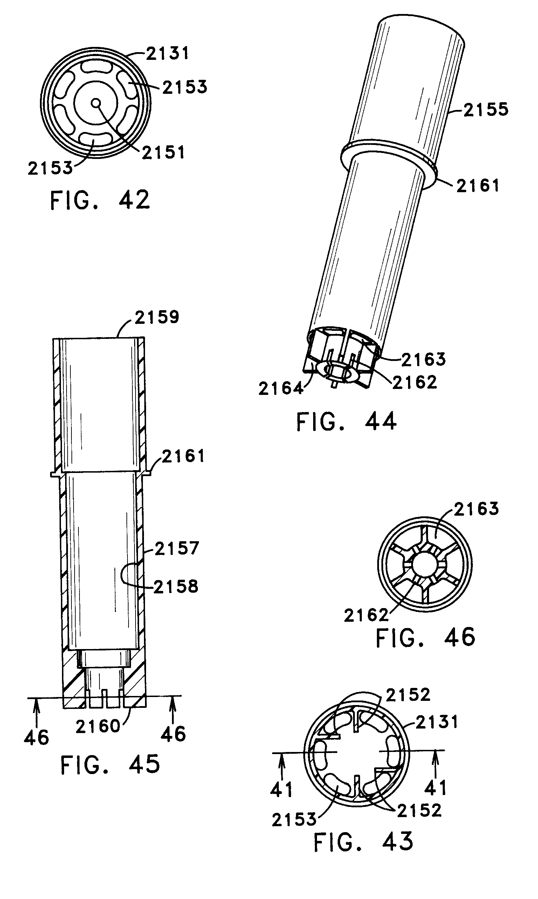

Referring to Figure 44 in conjunction with Figure 45, Figure 46 and Figure 47 the assembly 2128 also includes a carrier 2155 and a test strip 2156. The carrier 2155 is hollow and substantially cylindrical. The carrier 2155 has an outer surface 2157, an inner surface 2158, an open end 2159 and a closed end 2160. The carrier 2155 also has a flange 2161, four slots 2162 and six holes 2163. The flange 2161 disposed on the outer surface 2157. The four slots 2162 and the six holes 2163 are disposed on the outer surface 2157 at the closed end 2160. The carrier 2155 is inserted into the sleeve 2131, six chambers 2164 are formed therebetween. The four slots 2162 guide the rotational positioning of the carrier 2155 and engage the four alignment bars 2152 of the sleeve 2131 so that each of the six holes 2163 of the carrier 2155 is aligned with one of the six holes 2153 of the sleeve 2131 in order to form one of six first passageways 2165. The sleeves 2155 has six second passageways 2166.

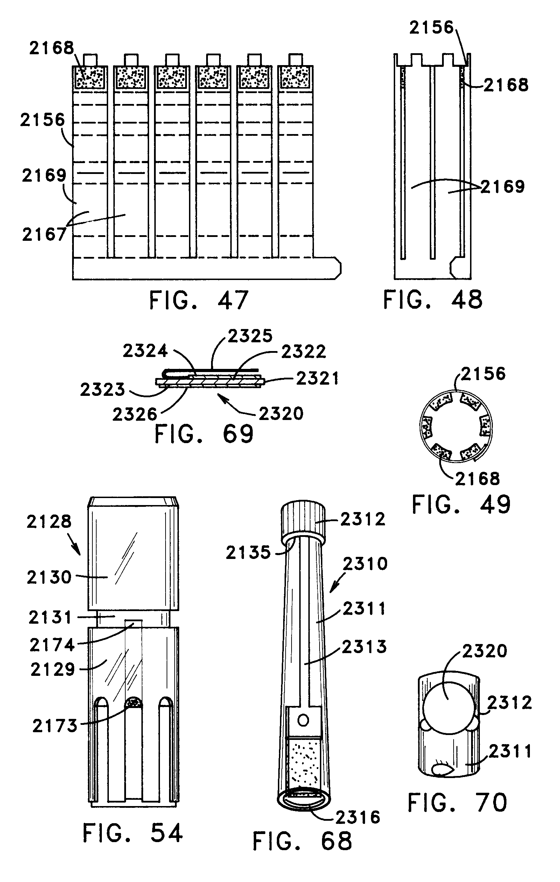

Referring to Figure 48 in conjunction with Figure 47 and Figure 49 the test strip 2156 has six segments 2167. Each segment 2167 has an absorbing pad 2168 and a reagent strip 2169. The absorbing pad 2168 contains dry reagents for use in enzyme immunoassay (EIA) tests. U. S. Patent No. 4,169,012 teaches peroxidase compositions which contain polyvalent ions of groups III and IV of the Periodic Table. These peroxidase compositions are stable even in low concentrations and are relatively invulnerable to freeze-drying. The polyvalent ions selected are those of Al, Zn, Mg, Fe, and Cu. The reagent strip 2169 is mechanically and fluidly coupled to the absorbing pad 2168. The reagent strip 2169 of each segment 2167 of the test strip 2156 is inserted between the carrier 2155 and the sleeve 2131. Each absorbing pad 2168 is disposed in one of the six chambers 2164. Each first passageway 2165 fluidly couples the sample container 2111 to one of the six chambers 2164.

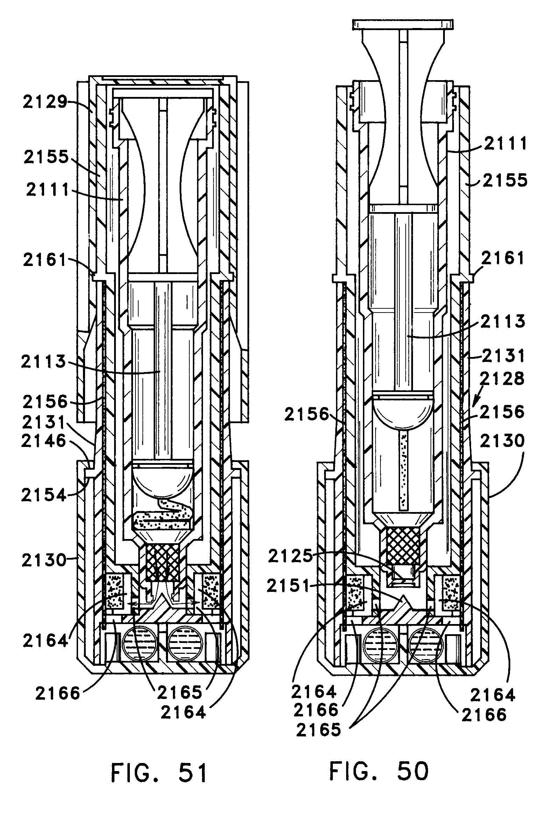

Referring to Figure 50 when an adequate amount of sample is collected, the sample collector 2113 is inserted into the sample container 2111 in order to mix the buffering solution 2126 and the sample together. The sample container 2111 is inserted into the carrier 2155 of the assembly 2128.

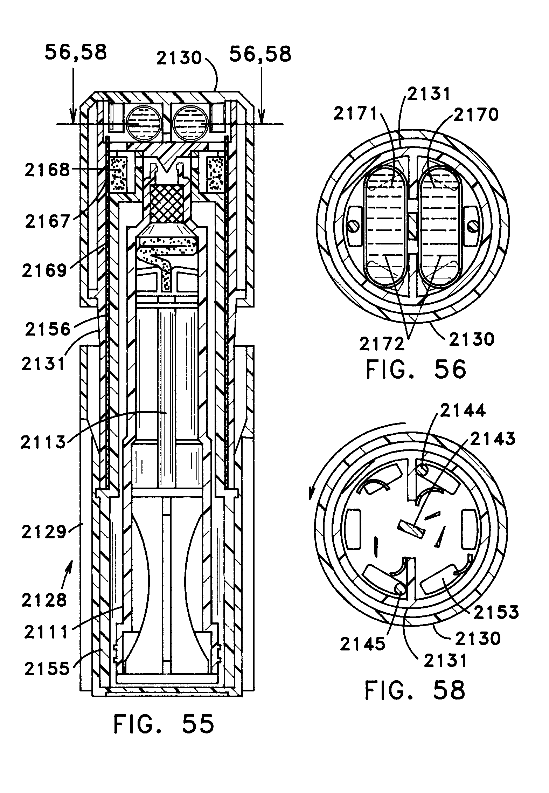

Referring to Figure 51 in conjunction with Figure 52 and Figure 53 when the sample container 2111, the sample collector 2113 and the assembly 2128 are put together, the flange 2160 of the carrier 2155 engages the locking notch 2138 of the lower cap 2129 in order to secure the lower cap 2129 to the carrier 2155 so that the sample container 2111 and the sample collector 2113 can not be removed from the carrier 2155. The flange 2154 of the sleeve 2131 engages the inner retaining ridge 2146 of the upper cap 2130 in order to secure the upper cap 2130 to the sleeve 2131. When the sample collector 2113 is pressed downward the seal-breaker 2151 breaks breakable seal 2125 of the sample container 2111 thereby releasing the mixture of the buffering solution 2126 and the sample so that this mixture flows into the chamber 2164 through the filter 2127.

Referring to Figure 54 in conjunction with Figure 55, Figure 56 and Figure 57, after the sample collector 2113 and the sample container 2111 have been pressed downward into the assembly 2128, the sample collector 2113, the sample container 2111 and the assembly 2128 are turned over so that the mixture of the buffering solution 2126 and the sample flow onto the absorbing pad 2168 of segments 2167 of test strip 2156.

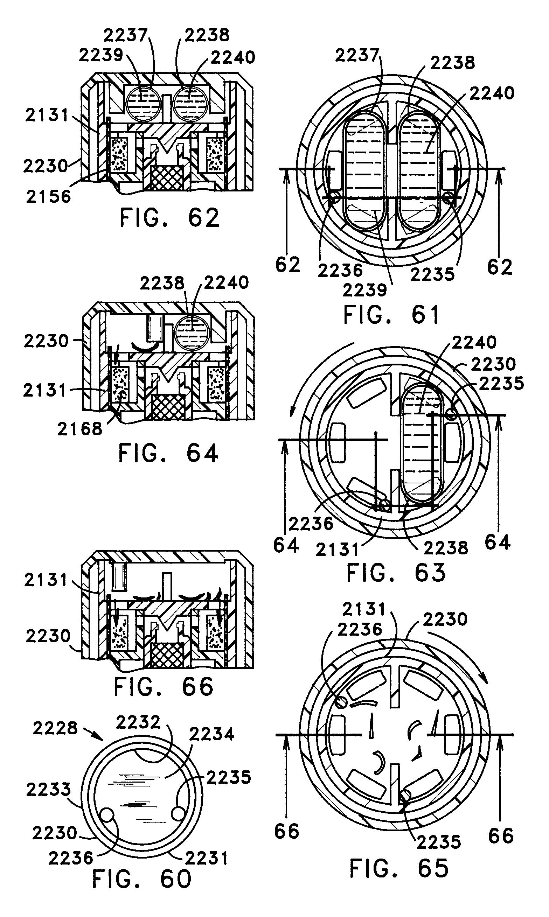

Referring to Figure 58 in conjunction with Figure 56 and Figure 59 the upper cap 2130 has a first capsule 2170 and a second capsule 2171. The first and second capsules 2170 and 2171 are glass ampules and contain a developer liquid 2172. Each second passageway 2166 fluidly couples the first and second capsules 2170 and 2171 to one of the six chambers 2164. After the mixture of the buffering solution 2126 and the sample has flowed into the chamber 2164 through the first passageway 2165 onto the absorbing pad 2168 of each segment 2167 of the test strip segment 2156, the upper cap 2130 is turned counter-clockwise in order to break the first and second capsules 2170 and 2171 so that the developer liquid 2172 flows into the chamber 2164 through the second passageway onto the absorbing pad 2168 of the segments 2167 of the test strip 2156 and the developer liquid 2172 interacts with the mixture of the buffering solution 2126 and the sample on each segment 2167. The reagent strip 2169 of each segment 2167 of the test strip 2156 has a sample validity area 2173 and a test result area 2174.

Referring to Figure 60 in conjunction with Figure 61 and Figure 62 an assembly 2228 includes an upper cap 2230 which is hollow and substantially cylindrical. The upper cap 2230 has an outer surface 2231, an inner surface 2232, an open end 2233 and a closed end 2234. The upper cap 2230 also has a first post 2235 and a second post 2236. The first and second posts 2235 and 2236 are oppositely disposed on the inner surface 2232 of the closed end 2234. The upper cap 2230 has a first capsule 2237 and a second capsule 2238. The first and second capsule 2237 and 2238 are glass ampules and contain a first developer liquid 2239 and a second developer liquid 2240, respectively.

Referring to Figure 63 in conjunction with Figure 64, after the sample container 2111, the sample collector 2113 and the assembly 2228 have been turned over, the upper cap 2230 is rotated counter-clockwise to break the first capsule 2237 in order to allow the first developer liquid 2239 to flow onto the absorbing pad 2168 of each segment 2167 of the test strip 2156 so that the first liquid developer 2239 interacts with the mixture of the buffering solution 2126 and the sample on each segment 2167.

Referring to Figure 65 in conjunction with Figure 66 after the first capsule 2237 has been broken the upper cap 2230 is rotated clockwise to break the second capsule 2238 in order to allow the second developer liquid 2240 to flow onto the absorbing pad 2168 of each segment 2167 of the test strip 2156 so that the second liquid developer interacts with the mixture of the buffering solution 2126, the sample and the first developer liquid 2239 on each segment 2167.

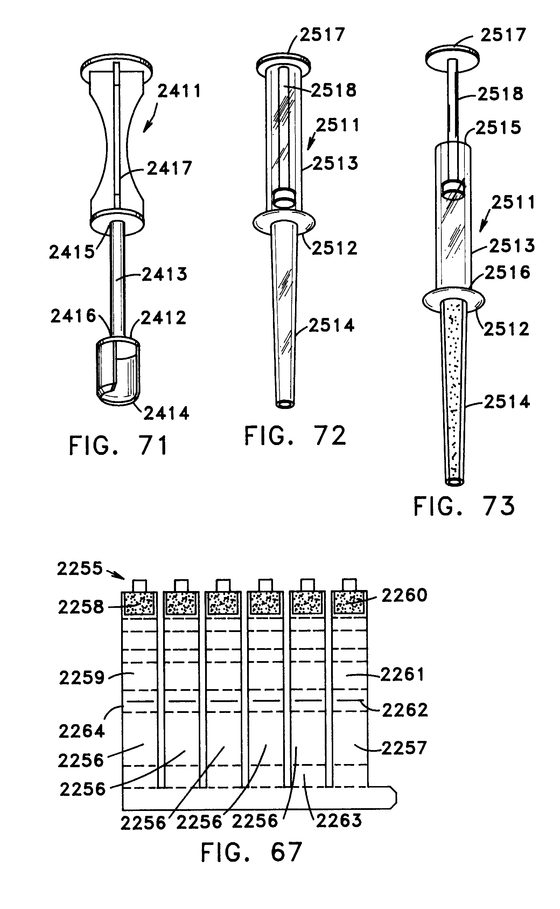

Referring to Figure 67 in conjunction with Figure 63 a test strip 2255 has five testing segments 2256 and one timing segment 2257. Each testing segment 2256 has an absorbing pad 2258 and a reagent strip 2259. The absorbing pad 2258 contains dry reagents for use in enzyme immunoassay (EIA) tests. The reagent strip 2259 is mechanically and fluidly coupled to the absorbing pad 2258. Each timing segment 2257 has an absorbing pad 2260 and a timing strip 2261 which has a drop of dye 2262 which interacts with the first developer liquid 2239. The reagent strips 2259 and the timing strip 2261 are inserted between the carrier 2155 and the sleeve 2131. The absorbing pad 2258 of each testing segment 2256 is disposed in one of the five of the six chambers 2164. The absorbing pad 2260 of the timing segment 2257 is disposed in other chamber 2164.

Referring to Figure 67 in conjunction with Figure 65 the dye 2262 changes colors when it interacts with the first developer liquid 2239. The dye 2262 can be chosen so that the time from when the first developer liquid 2239 contacts the dye 2262 to the time when the first developer liquid 2239 has interacted sufficiently enough with the mixture of the buffering solution 2126, the sample and the first developer liquid 2239 to insure a valid test is controlled. Once the dye 2262 has changed colors the upper cap 2230 is rotated clockwise to break the second capsule 2238 in order to allow the second developer liquid 2240 to flow onto the absorbing pad 2258 of each test segment 2256 of the test strip 2255 so that the second liquid developer interacts with the mixture of the buffering solution 2126, the sample and the first developer liquid 2239 on each testing segment 2257. The reagent strip 2259 of each test segment 2256 of the test strip 2255 has a sample validity area 2263 and a test result area 2264.

Referring to Figure 68 a sample sampling device 2310 includes a sample container 2311, a cap 2312 and a sample collector 2313. The sample container 2311 has an open end 2315 and a closed end 2316. The cap 2312 is coupled to the open end 2315 of the sample container 2311.

Referring to Figure 69 a fingerprint sensitive pad 2320 includes a substrate sheet 2321 which has a reflective surface 2322 and an opposing surface 2323. The fingerprint sensitive pad 2320 also includes a layer 2324 of inkless imprinting material, a flexible protective cover 2325 and a layer 2326 of adhesive backing. The layer 2324 of inkless imprinting material is applied to the reflective surface 2322 in order to receive an imprint of a finger. The flexible protective cover 2325 is hingedly mounted along an edge of the substrate sheet 2321 in order to protect a latent fingerprint image from degradation and adulteration during storage. The layer 2326 of adhesive backing is applied to the opposing surface in order to secure the fingerprint sensitive pad 2320 to a document so that the document in association with the fingerprint sensitive pad 2320 provides an identification records system for positively identifying a person involved in a transaction. U. S. Patent No. 4,943,089 teaches a fingerprint sensitive pad.

Referring to Figure 70 in conjunction with Figure 68 and Figure 69 when a sample has been collected from a person and the sample collector placed in the sample container 2311, the cap 2312 is coupled to the sample container 2311 in order to seal the sample container 2311 air-tight. The person presses his finger on the layer 2324 of inkless imprinting material of the fingerprint sensitive pad 2320. The layer 2326 of adhesive backing secures the fingerprint sensitive pad 2320 to both the cap 2312 and the sample container 2311. The imprint of his finger identifies the person from whom the sample was received. The use of fingerprint sensitive pad 2320 prevents any tampering with the received sample.

Referring to Figure 71 a sample collector 2411 of a sampling device for collecting either stool or soil. The sample collector 2411 includes a piston 2412, an elongated member 2413 and a scoop 2414. The elongated member 2413 has a first end 2415 and a second end 2416. The first end 2415 of the elongated member 2413 is coupled to a push tab 2417 The piston 2412 is coupled to the second end 2416 of the elongated member 2413. The scoop 2414 is mechanically coupled to the piston 2412 so that a technician can collect a sample without touching the sample. U. S. Patent No. 4,809,790 teaches a device for extracting an undisturbed sample of soil to be tested from a subsurface location uses a cutter to remove unwanted soil from above the undisturbed sample. The device is particularly intended for extraction of soil from contaminated areas without loss of contaminants which are volatile at ambient temperatures.

Figure 72 in conjunction with Figure 73 a sample collector 2511 of a sampling device for collecting blood. The sample collector 2511 includes a piston 2512, a hollow cylinder 2513 and a hollow, tapered cylinder 2514. The hollow cylinder 2513 has a first end 2515 and a second end 2516. The first end 2515 of the hollow cylinder 2513 is coupled to a push tab 2517 The piston 2512 is slidably coupled within the hollow cylinder 2513. The piston 2512 is mechanically coupled to a plunger 2518. A technician collects a sample of blood by drawing back the plunger 2518 and discharges the sample of blood into the sample container 2111 by pressing downward on the plunger 2518.

From the foregoing it can be seen that saliva sampling devices have been described. It should be noted that the sketches are not drawn to scale and that distance of and between the figures are not to be considered significant.

Accordingly it is intended that the foregoing disclosure and showing made in the drawing shall be considered only as an illustration of the principle of the present invention.

**Claims**

1. A sampling device comprising:
   a. a sample container having a first opening at a first end, a second end spaced from said first opening and a passageway through said second end of said sample container;
   b. a collection container having a second opening, said second opening being sized so that said second end of said sample container fits within said second opening of said collection container;

   c. a selective closure in said passageway; and
   d. a sample collector sized to fit through said first opening of said sample container.

2. A sampling device according to claim 1, wherein said collection container has a base and said sampling device also comprises:
   a. a test pad disposed on said base; and,
   b. a support having at least one hole disposed in said collection container beneath said first opening and above said base.

3. A sampling device according to Claim 1, wherein said collection container includes a base; a test strip; and, at least one reagent container used in mixing a sample with at least one reagent solution.

4. A sampling device according to Claims 2 or 3, wherein said selective closure includes a closure element blocking said passageway.

5. A sampling device according to Claim 4, wherein said collection container includes an actuator element extending away from said base, whereby said actuator element displaces said closure element to open said passageway when said second end of said sample container is inserted into said second opening of said collection container.

6. A sampling device according to claim 1 wherein said sample collector includes a porous mass and a rigid handle, said rigid handle extending to said passageway when inserted into said sample container to squeeze said porous mass at said passageway.

7. A sampling device according to claim 6 wherein said handle includes a piston closely fitting within said sample container to seal therebetween.

8. A sampling device according to claim 7 wherein said sample container includes an inner wall surface and a retaining ridge disposed on said inner wall surface, said retaining ridge being adapted to secure said piston of said collector within said sample container.

9. A sampling device comprising:
   a. a sample container having a first opening at a first end, a second end spaced from said first opening and a passageway through said second end of said sample container;
   b. a collection container having a second opening and a base, said second opening being sized so that said second end of said sample container fits within said second opening of said collection container;

c. a sample collector sized to fit through said first opening of said sample container; and,

d. a test pad disposed on said base of said collection container.

10. A sampling device comprising:

a. a sample container having a first opening at a first end, a second end spaced from said first opening and a passageway through said second end of said sample container;

b. a collection container having a second opening and a base, said second opening being sized so that said second end of said sample container fit within said second opening of said collection container;

c. a closure element blocking said passageway;

d. an actuator element extending from said collection container upwardly to said passageway of said sample container to contact and interfere with upon insertion of said sample container in said collection container to displace said closure element and open said passageway.

e. a sample collector sized to fit through said first opening of said sample container a portion of the length thereof for collecting a sample of biological fluids;

f. a test pad disposed on said base of said collection container; and,

g. a solution container containing a chemical solution for use in mixing the collected sample of biological fluids with the chemical solution.

11. A sampling device comprising:

a. a sample collector which selectively receives a sample of biological fluid; and,

b. an indicator activated by a preselected amount of the received sample of biological fluid to indicate that the collected sample of biological fluid is of adequate amount.

12. A sampling device according to claim 11 wherein said indicator comprises:

a. a cylinder having an open end and a closed end;

b. a solution contained in said cylinder; and,

c. a label having a bottom edge attached to said cylinder, said bottom edge of said label being disposed adjacent to the liquid level of said solution whereby, when said sample collector is placed in said cylinder, if the liquid level of said solution does not drop below said bottom edge of said label then the collected sample is of an adequate amount.

13. A sampling device according to Claim 1, including means to secure said sample container to said collection container.

14. A sampling device according to Claim 1, including means to seal said first opening of said sample container.

15. A sampling device according to Claim 1, wherein said sample container contains a chemical solution therein.

16. A sampling device according to claim 15, wherein said sample container includes a breakable seal, said breakable seal being at a location between said first end and said second end, said buffering solution being between said second end and said breakable seal.

17. A sampling device according to Claim 1, wherein said sample collector includes a fingerprint sensitive pad.

FIG. 1

FIG. 2

FIG. 3

FIG. 4

FIG. 5

FIG. 6

FIG. 7

FIG. 8

FIG. 10

FIG. 11

FIG. 9

FIG. 12

FIG. 13

FIG. 14

FIG. 15

FIG. 16

FIG. 17

FIG. 18

FIG. 19

FIG. 20

FIG. 21

FIG. 22

FIG. 24

FIG. 25

FIG. 23

FIG. 26

FIG. 27

FIG. 28

FIG. 29

FIG. 30

FIG. 53

FIG. 57

FIG. 31

FIG. 59

2128
2130
2131
2136
2129
2137

FIG. 32

2134
2136
2129
2133
2132
2135

FIG. 33

2138   2161   2131
2155
2129
2133

FIG. 52

2134
FIG. 52
2136
2138
36   36
2137
2133   2132
2135

FIG. 34

2137
2129
2135
2133   2132

FIG. 35

2132
2129
2137
2135
2133

FIG. 36

2142

2130

2139

2141

FIG. 37

2143    2142

2145    2144

39    39

2140

2130

2139

2146    2141

FIG. 38

2143    2130

2145

2142    2144

FIG. 39

2131

2154

FIG. 40

43    43

2150

2151

2131

2154

2147

2148    2148

2149

FIG. 41

2131

2153

2151

2153

FIG. 42

2155

2161

2163

2162

2164

FIG. 44

2159

2161

2157

2158

46

2160

46

FIG. 45

2163

2162

FIG. 46

2152

2131

41

41

2153

2152

FIG. 43

FIG. 47

FIG. 48

FIG. 69

FIG. 49

FIG. 54

FIG. 68

FIG. 70

FIG. 51

FIG. 50

FIG. 55

FIG. 56

FIG. 58

FIG. 62

FIG. 61

FIG. 64

FIG. 63

FIG. 66

FIG. 60

FIG. 65

FIG. 71

FIG. 72

FIG. 73

FIG. 67